# EUROPEAN PATENT SPECIFICATION

(11) **EP 1 674 448 B1**
(45) Date of publication and mention of the grant of the patent: **17.10.2007**
(21) Application number: 05027958.7
(22) Date of filing: 20.12.2005
(51) Int. Cl.: C07C 249/04, C07C 251/44

(54) **Process for producing cyclohexanone oxime**
Verfahren zur Herstellung von Cyclohexanonoxim
Procédé de préparation de l'oxime de la cyclohexanone

(30) Priority: 22.12.2004 JP 2004370726
(43) Date of publication of application: 28.06.2006
(73) Proprietor: Sumitomo Chemical Company, Limited, Tokyo 104-8260 (JP)
(72) Inventor: Trivellone, Franco, 20052 Monza (IT); Furlan, Piero, 31100 Treviso (IT); Fukao, Masami, Ritto-shi Shiga-ken (JP)
(74) Representative: Vossius & Partner

(56) References cited:
- EP-A- 0 005 291
- EP-A- 0 496 385
- EP-A- 1 270 548

## Description

The present invention relates to a process for producing cyclohexanone oxime. Cyclohexanone oxime is useful as a raw material of ε-caprolactam and further, a raw material of nylon-6, and the like.

As a process for producing cyclohexanone oxime, a process wherein an ammoximation reaction of cyclohexanone is carried out with hydrogen peroxide and ammonia in the presence of a titanosilicate catalyst has been known (see for example, JP-A-62-59256 (1987), JP-A-6-49015 (1994) and JP-A-6-92922 (1994)). With respect to a process for collecting cyclohexanone oxime from a reaction solution which has been obtained by the ammoximation reaction, for example, JP-A-62-59256 (1987) discloses a process wherein the above-mentioned ammoximation reaction is carried out using water as a reaction solvent and cyclohexanone oxime is extracted with toluene from the reaction solution after removing the catalyst from the solution by filtration. Further, JP-A-6-92922 (1994) discloses a process which includes carrying out the above-mentioned ammoximation reaction using a mixed solvent of t-butyl alcohol and water as the reaction solvent, subjecting the reaction solution from which the catalyst has been removed by filtration to distillation to remove unreacted ammonia and water-containing t-butyl alcohol as distillate, and extracting cyclohexanone oxime with toluene from the bottom product, and then subjecting the extract to distillation to remove toluene as distillate, and collecting cyclohexanone oxime as a bottom product. Further, EP-A-1270548 describes a purification method of cyclohexanone oxime in a solution of solvents immiscible, with water, which consists in washing said solutions with water or with an aqueous solution of a base having a pK <5 or in passing the solutions of cyclohexanone oxime through a weakly alkaline ion exchange resin.

However, the thermal stability of cyclohexanone oxime which is collected according to the above-mentioned conventional processes is not always adequate. For this reason, there are a problem caused by generation of a tar and problems of reduction in yield and deterioration in quality, for example, when the collected cyclohexanone oxime is stored and transferred in a heated and melted state, and when the collected cyclohexanone oxime is gasified for purification by distillation or for the Beckmann rearrangement reaction.

Then, the present inventors have intensively studied for the purpose of developing a process for advantageously collecting cyclohexanone oxime superior in thermal stability, in a process for producing cyclohexanone oxime by the above-mentioned ammoximation reaction. As a result, the inventors have found that the above-mentioned purpose can be attained by carrying out the ammoximation reaction and then subjecting said reaction solution to a series of steps consisting of predetermined distillation, extraction and washing, to complete the present invention.

The present invention is to provide a process for producing cyclohexanone oxime which includes the following steps (1) to (6);
(1) a step of reacting cyclohexanone, hydrogen peroxide and ammonia in the presence of a titanosilicate catalyst to give a reaction solution containing cyclohexanone oxime, water, unreacted ammonia and unreacted cyclohexanone (hereinafter, this step is referred to as a "reaction step"),
(2) a step of subjecting the reaction solution obtained in the step (1) to distillation to distil off ammonia and obtain a bottom product containing cyclohexanone oxime, water and cyclohexanone (hereinafter, this step is referred to as a "first distillation step"),
(3) a step of mixing the bottom product obtained in the step (2) with an organic solvent followed by separating a mixture into an organic layer and an aqueous layer (hereinafter, this step is referred to as an "extraction step"),
(4) a step of mixing the organic layer obtained in the step (3) with water followed by separating a mixture into an organic layer and an aqueous layer (hereinafter, this step is referred to as a "washing step"),
(5) a step of subjecting the organic layer obtained in the step (4) to distillation to distill off the organic solvent and water and to obtain a bottom product containing cyclohexanone oxime and cyclohexanone (hereinafter, this step is referred to as a "second distillation step"), and
(6) a step of subjecting the bottom product obtained in the step (5) to distillation to distil off cyclohexanone and to obtain a bottom product containing cyclohexanone oxime (hereinafter, this step is referred to as a "third distillation step").

In this production process, the ammonia distilled off in the first distillation step (2) is desirably recycled as ammonia used in the reaction step (1). Further, the organic solvent distilled off in the second distillation step (5) is desirably recycled as the organic solvent used in the extraction step (3), and the water distilled off in the second distillation step (5) is desirably recycled as water used in the washing step (4). Furthermore, the cyclohexanone distilled off in the third distillation step (6) is desirably recycled as cyclohexanone used in the reaction step (1).

Additionally, it is desirable that the washing step (4) includes a first washing step (4a) wherein the organic layer obtained in the extraction step (3) is mixed with an aqueous solution containing a base and then a mixture is separated into an organic layer and an aqueous layer, and a second washing step (4b) wherein the organic layer obtained in the first washing step (4a) is mixed with water not substantially containing a base and then a mixture is separated into an organic layer and an aqueous layer. Here, a mixture of a base and the aqueous layer that is obtained in the second washing step (4b) may be recycled as the aqueous solution containing a base which is used in the first washing step (4a), and the water distilled off in the second distillation step (5) may be recycled as the water not substantially containing a base which is used in the second washing step (4b).

According to the present invention, cyclohexanone oxime superior in thermal stability can be advantageously produced.

The process for producing cyclohexanone oxime of the present invention consists of a series of steps which include (1) a reaction step wherein the ammoximation reaction of cyclohexanone with hydrogen peroxide and ammonia is carried out in the presence of a titanosilicate catalyst to give a reaction solution, (2) a first distillation step wherein residual ammonia is distilled off from the reaction solution and a cyclohexanone oxime/water-mixed solution (including unreacted cyclohexanone) is obtained as a bottom product, (3) an extraction step wherein cyclohexanone oxime (including unreacted cyclohexanone) is extracted with an organic solvent from the cyclohexanone oxime/water-mixed solution, (4) a washing step wherein an extract containing cyclohexanone oxime is washed with water, (5) a second distillation step wherein the organic solvent is distilled off from the extract containing cyclohexanone oxime which has been washed, to obtain crude cyclohexanone oxime, and (6) a third distillation step wherein residual cyclohexanone is distilled off from the crude cyclohexanone oxime and pure cyclohexanone oxime is obtained as a bottom product. All of these steps may be carried out in a continuous operation. Alternatively, a part of the steps may be carried out in a continuous operation and the other part of them may be carried out in batch operations. Alternatively, all of them may be carried out in batch operations. However, it is preferable that all steps are carried out in a continuous operation from the viewpoints of productivity of cyclohexanone oxime and the thermal stability and quality of cyclohexanone oxime obtained by this process.

In the embodiment of a continuous production process of cyclohexanone oxime according to the present invention, firstly, a predetermined amount of' the reaction solution with the catalyst dispersed therein is held in a reaction vessel 1. The ammoximation reaction is conducted by feeding cyclohexanone 11, hydrogen peroxide 12, ammonia 13, and a solvent if necessary in the reaction solution while drawing the reaction solution 14 whose amount is substantially equal to those of the raw materials (reaction step (1)). Here, the reaction solution 14 is preferably drawn such that only its liquid phase is drawn through a filter or the like, and the catalyst remains in the reaction vessel 1. When the catalyst is simultaneously drawn, a step of separating the catalyst from the drawn reaction solution 14 is required and the catalyst is required to be fed to the reaction vessel 1. The concentration of the catalyst depends on the activity, reaction conditions and the like, and is usually within a range of 1g/L to 200g/L, when the concentration is indicated as the weight per the volume of the reaction solution (catalyst + liquid phase). Further, the reaction vessel 1 is preferably glass-lined one or made of stainless steel from the viewpoint of prevention of the decomposition of hydrogen peroxide.

The amount of hydrogen peroxide is usually from 0.5 to 3-fold by mol, and preferably from 0.5 to 1.5-fold by mol based on the moles of cyclohexanone. Further, the amount of ammonia is usually 1-fold by mol or more and preferably 1.5-fold by mol or more based on the moles of cyclohexanone. Ammonia is used in a more excessive amount than that of cyclohexanone and hydrogen peroxide so as to reside in the reaction solution and thereby, the conversion of cyclohexanone and the selectivity of cyclohexanone oxime are enhanced.

Preferable solvents used in the reaction step (1) include, for example, alcohols such as methyl alcohol, ethyl alcohol, n-propyl alcohol, isopropyl alcohol, n-butyl alcohol, sec-butyl alcohol, tert-butyl alcohol and tert-amyl alcohol, water and a mixed solvent thereof.

The reaction temperature is usually within a range of 50°C to 120°C, and preferably within a range of 70°C to 100°C. Further, the reaction is preferably carried out under pressure for enhancing the solubility of ammonia in the reaction solution.

In the reaction solution 14 thus obtained, water which is produced by the reaction is theoretically contained in an amount of 2-fold by mol based on the moles of cyclohexanone, together with the objective cyclohexanone oxime (C₆H₁₀O + NH₃ + H₂O₂ → C₆H₁₀NOH + 2H₂O). Further, since hydrogen peroxide is usually used in a form of a 10-70wt% H₂O₂ containing aqueous solution, the water in this solution is also contained in the reaction solution 14. Further, since ammonia is excessively used as described above, unreacted ammonia is also contained in the reaction solution 14. Furthermore, since it is difficult for cyclohexanone to completely react, unreacted cyclohexanone is also contained in the reaction solution 14.

Next, the reaction solution 14 containing cyclohexanone oxime, water, ammonia and cyclohexanone is introduced in a first distillation column 2 to be subjected to distillation, ammonia is collected as a distillate 15, and a mixture of cyclohexanone oxime, water and cyclohexanone is obtained as a bottom product 16 (the first distillation step 2). The distillation is usually carried out under normal pressure and may be carried out under pressure or under reduced pressure, if necessary.

Ammonia collected as the distillate 15 is desirably recycled to the reaction vessel 1. Further, when an organic solvent is used in the reaction step (1), that is, when an organic solvent is fed to the reaction vessel 1, the organic solvent is contained in the reaction solution 14 and introduced into the first distillation column 2. It is desirable that also this organic solvent is collected as the distillate 15 and recycled to the reaction vessel 1. The organic solvent used in the reaction step (1) is required to have a lower boiling point than that of cyclohexanone oxime in order to recycle also the organic solvent to the reaction vessel 1. Some of organic solvents are distilled off accompanying water as an azeotrope or the like. In that case, the water may be also recycled together with the organic solvent to the reaction vessel 1. For example, since tert-butyl alcohol is distilled off as water-containing tert-butyl alcohol containing water in an amount of 12% to 18% by weight, the water-containing t-butyl alcohol may be recycled to the reaction vessel 1. Ammonia is usually recycled to the reaction vessel 1 in a gaseous form using a compressor. Alternatively, ammonia may be recycled to the reaction vessel 1 as a solution by being cooled to a low temperature under pressure and dissolved in an organic solvent or an organic solvent containing water.

The bottom product 16 drawn from the first distillation column 2 is introduced in an extractor 3 followed by being mixed with an organic solvent 17, and then the mixture is separated into an organic layer 18 containing cyclohexanone oxime and cyclohexanone which are extracted from the bottom product 16 with the organic solvent and an aqueous layer 19 which is a raffinate (the extraction step (3)). The organic solvent 17 for extraction is required to be able to be separated from water, to have capability of dissolving cyclohexanone oxime and to have a lower boiling point than that of cyclohexanone oxime. Suitable examples of the organic solvents include an aromatic hydrocarbon solvent such as toluene, an alicyclic hydrocarbon solvent such as cyclohexane, an aliphatic hydrocarbon solvent such as hexane and heptane, an ether such as diisopropyl ether and tert-butylmethyl ether, an ester such as ethyl acetate and the like. The hydrocarbon solvents such as toluene, cyclohexane and heptane are preferable and the aromatic hydrocarbon solvent such as toluene is particularly preferable. The amount of the organic solvent 17 is usually from 0.1 to 2-fold by weight, and preferably from 0.3 to 1-fold by weight, based on the amount of cyclohexanone oxime in the bottom product 16. The extraction temperature is usually selected from a range of a normal temperature to the boiling point of the organic solvent 17 and is preferably within a range of 40°C to 90°C. The extractor 3 may be one by which multi-stage extraction can be carried out, or a single-stage or multi-stage mixer settler type extractor in which a mixing portion and a liquid/liquid-separation portion are separated.

The organic layer 18 from the extractor 3 is introduced in a washer 4 followed by being mixed with water 20, and then a mixture is separated into an organic layer 21 which is obtained by removing impurities from the organic layer 18 by washing with water and an aqueous layer 22 in which the impurities are dissolved (the washing step (4)). The impurities include, for example, ammonium nitrate, ammonium nitrite and the like which are yielded as by-products in the reaction step (1). The amount of water 20 used for washing is usually from 0.05 to 1-fold by weight based on the weight of cyclohexanone oxime in the organic layer 18, and the washing temperature is usually within a range of 40°C to 90°C. Further, the washer 4 may be one by which multi-stages extraction can be carried out, or a single-stage or multi-stage mixer settler type extractor in which a mixing portion and a liquid/liquid-separation portion are separated, similarly to the extractor 3.

The organic layer 21 from the washer 4 is introduced into a second distillation column 5 to be subjected to distillation, such that the organic solvent in the organic layer 21 can be collected as a distillate 23 and the crude cyclohexanone oxime containing the above-mentioned unreacted cyclohexanone can be obtained as the bottom product 24 (the second distillation step (5)). In this step, water dissolved and/or dispersed in the organic layer 21 is collected as the distillate 23 together with the organic solvent. The distillation is usually carried out at a pressure between a normal pressure and a slightly reduced pressure depending on the boiling point of the organic solvent.

It is desirable that the organic solvent and water which are collected as the distillate 23 are recycled as the organic solvent 17 for extraction which is introduced in the extractor 3 and water 20 for washing which is introduced in the washer 4, respectively. Specifically, the organic solvent and water which are collected as the distillate 23 are introduced in a liquid/liquid separator 7 to be separated into the organic layer 17 and the aqueous layer 20, and then the organic layer 17 is introduced in the extractor 3, and the aqueous layer 20 is introduced in the washer 4.

The crude cyclohexanone oxime which has been drawn as the bottom product 24 from the second distillation column 5 is introduced into a third distillation column 6 to be subjected to distillation such that the cyclohexanone can be collected as a distillate 25 and purified cyclohexanone oxime which contains no or reduced amount of cyclohexanone can be obtained as a bottom product 26 (the third distillation step (6)). The distillation is usually carried out at a temperature of 140°C or less under reduced pressure of 10 kPa or less. Since the purified cyclohexanone oxime thus obtained is superior in thermal stability, it can be preferably used as a raw material for the gas phase Beckmann rearrangement. It is desirable that the distillation conditions are adjusted so that the concentration of cyclohexanone in said purified cyclohexanone oxime is 1% by weight or less and preferably 0.5% by weight or less from the viewpoint of suppressing a side reaction in the gas phase Beckmann rearrangement.

It is desirable to recycle cyclohexanone which is collected as the distillate 25 to the reaction vessel 1. Further, when the accumulation of impurities in the distillate 25 is remarkable because of operation for a long term and the like, at least a potion of the distillate may be purified by rectification or the like.

The washing step (4) may be modified for the purpose of further enhancing the thermal stability of purified cyclohexanone oxime which is obtained by the process as described above. Specifically, it is advantageous that the washing step (4) consists of a first washing step (4a) wherein an aqueous solution containing a base is used and a second washing step (4b) wherein water not substantially containing a base (so-called distilled water, deionized water, purified water or the like) is used. The amount of the aqueous layer which is generated in the whole washing step (4) (or the amount of water supplied from the outside of the process system) can be reduced by using a solution which is produced by mixing the water layer formed in the second washing step (4b) with a base, as the aqueous solution containing a base which is used in the first washing step (4a). Further, water which is the distillate of the second distillation step can be used as the water not substantially containing a base which is used in the second washing step (4b).

Further, it is desirable in order to reduce a loss of cyclohexanone oxime in the above process that cyclohexanone oxime which is contained in a very small amount in the aqueous layer discharged in the extraction step (3) and the aqueous layer discharged in the washing step (4) is extracted with an organic solvent to be collected. The organic solvent used here may be the same one as the organic solvent used in the extraction step (3). Accordingly, it is desirable that the organic solvent which is to be used in the extraction step (3) is firstly used for collecting a very small amount of cyclohexanone oxime from the above-mentioned aqueous layers, and then used in the extraction step (3). Further, the collection of cyclohexanone oxime is desirably carried out by multi-stage extraction.

In the embodiment of the above-mentioned two-stage washing accompanied by collection of cyclohexanone oxime, firstly, the two-stage washing is described. The organic layer 18 from the extractor 3 is introduced in a first washer 4a, mixed with an aqueous solution 20a containing a base and then separated into an organic layer 21a and an aqueous layer 22a [the first washing step (4a)]. The bases contained in the aqueous solution 20a include, for example, a hydroxide and a carbonate of an alkali metal, ammonia and the like. The concentration of the base in the aqueous solution 20a is usually within a range of 0.01N to 2N, and preferably within a range of 0.05N to 0.5N. The washing temperature is preferably within a range of 60°C to 90°C and the amount of the aqueous solution 20a containing a base is preferably from 0.2 to 2-fold by weight based on the weight of cyclohexanone oxime in the organic layer 18. Next, the organic layer 21a from the first washer 4a is introduced in a second washer 4b, mixed with water 20b not substantially containing a base and then separated into an organic layer 21b and an aqueous layer 22b [the second washing step (4b)]. Next, the organic layer 21b is introduced in the second distillation column 5 and subjected to distillation. Further, the aqueous layer 22b is mixed with a base or an aqueous solution of the base 27 and the mixed solution is recycled as the aqueous solution 20a containing a base which is introduced in the first washer 4a. The aqueous layer 20 obtained from the distillate 23 from the second distillation column 5 may be used as the water 20b not substantially containing a base which is introduced in the second washer 4b.

Next, the collection' of cyclohexanone oxime is described. The aqueous layer 19 from the extractor 3 and the aqueous layer 22a from the first washer 4a are introduced in a collector 8, mixed with the organic solvent 17, and separated into an organic layer 28 and an aqueous layer 29. The organic layer 28 contains a very small amount of cyclohexanone oxime which has been extracted from the water layer 19 and the aqueous layer 22a with the organic solvent 17. The aqueous layer 29 is a raffinate of extraction. The organic layer 28 is introduced in the extractor 3 and used as the organic solvent for extracting cyclohexanone oxime from the bottom product 16 of the first distillation column 2. Further, the aqueous layer 29 is treated as waste water. As the organic solvent 17 which is introduced in the collector 8, the organic layer 17 obtained from the distillate 23 from the second distillation column 5 may be used. Further, the collector 8 may be one by which multi-stage extraction can be carried out, or a single-stage or multi-stage mixer settler type extractor in which a mixing portion and a liquid/liquid-separation portion are separated, similarly to the extractor 3 and the washer 4.

Further, the aqueous layer discharged in the washing step (4) is advantageously subjected to the extraction step (3) together with the mixed solution of cyclohexanone oxime and water which is the bottom product of the first distillation step (2), in order to collect cyclohexanone oxime contained in a very small amount in the aqueous layer. In that case, the extraction step (3) is more advantageously carried out by multi-stage extraction, because the aqueous layer discharged from such an extraction step (3) can become an aqueous layer not substantially containing cyclohexanone oxime which is not required to be subjected to a collection treatment.

### EXAMPLES

The present invention is described by some examples, but it is not limited to these example. In Example, "%" representing the content is based on weight unless otherwise noticed.

### Example 1

The respective steps and thermal stability test were carried out by applying the above-mentioned two-stage washing accompanied by collection collection of cyclohexanone oxime to the continuous production process.

### [The reaction step (1)]

A reaction solution in which the titanosilicate catalyst was dispersed was hold in a stirring vessel-type reaction vessel 1, and reaction was carried out by drawing a reaction solution 14 through a filter while feeding cyclohexanone 11, 60% hydrogen peroxide aqueous solution 12, ammonia 13 and tert-butyl alcohol containing water in an amount of 15% by weight into the vessel. The reaction was carried out at 85°C and a pressure of 0.25 MPa (gauge pressure) under a feed molar ratio of cyclohexanone/hydrogen peroxide/ammonia/water/tert-butyl alcohol = 1/1.1/1.8/4.29/4.0. Nitrogen gas which was yielded as a by-product in the reaction was removed out of the system, and ammonia gas which accompanied the nitrogen gas was absorbed in water to be collected. The concentrations of components in the reaction solution 14 were 20.89% of cyclohexanone oxime, 21.69% of water, 2.30% of ammonia, 0.09% of cyclohexanone and 55.03% of tert-butyl alcohol.

### [The first distillation step (2)]

The reaction solution 14 which was drawn from the reaction vessel 1 was introduced into a first distillation column 2 together with the above-mentioned water wherein ammonia was absorbed, and subjected to distillation at a temperature of 83°C and a pressure of 50kPa (gauge pressure) such that ammonia and water-containing tert-butyl alcohol were collected as a distillate 15 and a mixture consisting of 46.23% of cyclohexanone oxime, 53.45% of water and 0.205% of cyclohexanone was obtained as a bottom product 16. The distillate 15 was used as a portion of ammonia which was fed to the reaction vessel 1 and the 15% water-containing t-butyl alcohol.

### [Extraction step (3)]

The bottom product 16 from the first distillation column 2 and toluene 28 whose weight was the same as that of cyclohexanone oxime contained in said bottom product 16 were introduced in an extractor 3, and the mixture was stirred at about 72°C and then separated into a toluene layer 18 and an aqueous layer 19.

### [The first washing step (4a)]

The toluene layer 18 from the extractor 3 and a 0.1N sodium hydroxide aqueous solution 20a whose amount was the same in weight as that of cyclohexanone oxime contained in said toluene layer 18 were introduced in a first washer 4a, and the mixture was stirred at about 72°C and then separated into a toluene layer 21a and an aqueous layer 22a.

### [The second washing step (4b)]

The toluene layer 21a from the first washer 4a and water 20b whose amount was the same in weight as that of cyclohexanone oxime contained in said toluene layer 21a were introduced in a second washer 4b, and the mixture was stirred at about 72°C and then separated with a decanter into a toluene layer 21b and an aqueous layer 22b. Sodium hydroxide was added to the aqueous layer 22b to prepare a 0.1N sodium hydroxide aqueous solution and this was used as the 0.1N sodium hydroxide aqueous solution 20a which was introduced in the first washer 4a.

### [Oxime collection step]

The aqueous layer 19 from the extractor 3 and the aqueous layer 22a from the first washer 4a were mixed, the mixed aqueous layer and toluene 17 were introduced in a collector 8. The mixture was stirred at about 72°C and then separated into a toluene layer 28 and an aqueous layer 29. The toluene layer 28 was used as toluene 28 which was introduced in the extractor 3.

### [The second distillation step (5)]

The toluene layer 21b from the second washer 4b was introduced into a second distillation column 5, subjected to distillation under reduced pressure of 30kPa at a temperature of 107°C and such that a mixture of toluene and water was collected as a distillate 23 and crude cyclohexanone oxime with a purity of 98.13% which contained 0.54% of cyclohexanone was obtained as the bottom product 24. The distillate 23 was introduced in a liquid-liquid separator 7 and separated into a toluene layer 17 and an aqueous layer 20 at about 35°C. The toluene layer 17 was used as the toluene 17 which was introduced in the collector 8 and the aqueous layer 20 was used as the water 20b which was introduced in the second washer 4b.

### [The third distillation step (6)]

The bottom product 24 from the second distillation column 5 was introduced into a third distillation column 6, and subjected to distillation under reduced pressure of 4kPa at a temperature of 120°C such that cyclohexanone was collected as a distillate 25 and purified cyclohexanone oxime with a purity of 99.70% which contained 0.02% of cyclohexanone was obtained as a bottom product 26. The distillate 25 was used as a portion of cyclohexanone which was fed to the reaction vessel 1.

### [Thermal stability test]

The purified cyclohexanone oxime which was obtained as the bottom product 26 was subjected to reduced pressure distillation at a temperature of 120°C and under a pressure of 15torr (2kPa) until the distillate was not observed. The amount of residual tar was 0.01% based on the charge fed to distillation (this tar amount corresponded to the amount of tar before heating treatment). Further, the cyclohexanone oxime was subjected to heating treatment at a temperature of 200°C under nitrogen gas flow for 5 hours, and then it was subjected to distillation under reduced pressure in the same manner as in the above-mentioned reduced pressure distillation. The amount of residual tar was 0.54% based on the charge fed to the distillation (this tar amount corresponded to the amount of tar after heating treatment). The increment of tar due to the heating treatment was 0.53%.

### Comparative Example 1

The reaction step (1) and the first distillation step (2) were carried out in the same manner as in Example 1. Ammonium sulfate was charged in the bottom product 16 from the first distillation column 2 in order to separate the bottom product into an organic solvent and an aqueous layer. The obtained organic layer was cyclohexanone oxime with a purity of 96.8% which contains 0.11% of cyclohexanone and 2.7% of water. The thermal stability test was carried out in the same manner as in Example 1 with respect to the cyclohexanone oxime obtained. The amount of tar was 0.21% before heating treatment, the amount of tar was 15% after heating treatment, and the increment of tar due to the heating treatment was 14.79%.

### Comparative Example 2

The reaction step (1), the first distillation step (2), the extraction step (3), the first washing step (4a) and the second washing step (4b) were carried out in the same manner as in Example 1. The toluene layer 21b from the second washer 4b was subjected to batch concentration under reduced pressure of 65kPa to 30kPa at a temperature of 104°C to 108°C over 16 hours to obtain cyclohexanone oxime with a purity of 99.1% which contains 0.66% of cyclohexanone as a bottom product. The thermal stability test was carried out in the same manner as in Example 1 with respect to cyclohexanone oxime obtained. The amount of tar was 0.07% before heating treatment, the amount of tar was 7.1% after heating treatment, and the increment of tar due to the heating treatment was 7.03%.

## Claims

1. A process for producing cyclohexanone oxime including the following steps (1) to (6):
(1) a step of reacting cyclohexanone, hydrogen peroxide and ammonia in the presence of a titanosilicate catalyst to give a reaction solution containing cyclohexanone oxime, water, unreacted ammonia and unreacted cyclohexanone,
(2) a step of subjecting the reaction solution obtained in step (1) to distillation to distil off ammonia and obtain a bottom product containing cyclohexanone oxime, water and cyclohexanone,
(3) a step of mixing the bottom product obtained in step (2) with an organic solvent followed by separating the mixture into an organic layer and an aqueous layer,
(4) a step of mixing the organic layer obtained in step (3) with water followed by separating the mixture into an organic layer and an aqueous layer,
(5) a step of subjecting the organic layer obtained in step (4) to distillation to distil off the organic solvent and water and to obtain a bottom product containing cyclohexanone oxime and cyclohexanone, and
(6) a step of subjecting the bottom product obtained in step (5) to distillation to distil off cyclohexanone and to obtain a bottom product containing cyclohexanone oxime.

2. The process according to claim 1, wherein the organic solvent used in step (3) is one or more solvents selected from a hydrocarbon solvent, an ether solvent, and an ester solvent.

3. The process according to claim 1 or 2, wherein the ammonia distilled off in step (2) is recycled to step (1).

4. The process according to any one of claims 1 to 3, wherein the organic solvent distilled off in step (5) is recycled to step (3).

5. The process according to any one of claims 1 to 4, wherein the water distilled off in step (5) is recycled to step (4).

6. The process according to any one of claims 1 to 5, wherein the cyclohexanone distilled off in step (6) is recycled to step (1).

7. The process according to any one of claims 1 to 6, wherein step (4) includes the following steps (4a) and (4b):
(4a) a step of mixing the organic layer obtained in step (3) with an aqueous solution containing a base followed by separating the mixture into an organic layer and an aqueous layer, and
(4b) a step of mixing the organic layer obtained in step (4a) with water not substantially containing a base followed by separating the mixture into an organic layer and an aqueous layer, and
the organic layer obtained in step (4b) is subjected to step (5).

8. The process according to claim 7, wherein the aqueous layer obtained in step (4b) is mixed with a base and the resulting aqueous solution containing a base is recycled to step (4a).

9. The process according to claim 7 or 8, wherein the water distilled off in step (5) is recycled to step (4b).

## Patentansprüche

1. Verfahren zur Herstellung von Cyclohexanonoxim umfassend die folgenden Schritte (1) bis (6):
(1) einen Schritt des Umsetzens von Cyclohexanon, Wasserstoffperoxid und Ammoniak in Gegenwart eines Titansilikatkatalysators, um eine Cyclohexanonoxim, Wasser, nicht umgesetzten Ammoniak und nicht umgesetztes Cyclohexanon enthaltende Reaktionslösung zu ergeben,
(2) einen Schritt, die in Schritt (1) erhaltene Reaktionslösung einer Destillation zu unterziehen, um Ammoniak abzudestillieren und ein Cyclohexanonoxim, Wasser und Cyclohexanon enthaltendes Sumpfprodukt zu erhalten,
(3) einen Schritt des Mischens des in Schritt (2) erhaltenen Sumpfprodukts mit einem organischen Lösungsmittel, gefolgt von Trennen des Gemisches in eine organische Schicht und eine wässrige Schicht,
(4) einen Schritt des Mischens der in Schritt (3) erhaltenen organischen Schicht mit Wasser, gefolgt von Trennen des Gemisches in eine organische Schicht und eine wässrige Schicht,
(5) einen Schritt, die in Schritt (4) erhaltene organische Schicht einer Destillation zu unterziehen, um das organische Lösungsmittel und Wasser abzudestillieren und ein Cyclohexanonoxim und Cyclohexanon enthaltendes Sumpfprodukt zu erhalten, und
(6) einen Schritt, das in Schritt (5) erhaltene Sumpfprodukt einer Destillation zu unterziehen, um Cyclohexanon abzudestillieren und ein Cyclohexanonoxim enthaltendes Sumpfprodukt zu erhalten.

2. Verfahren gemäß Anspruch 1, wobei das in Schritt (3) verwendete organische Lösungsmittel ein oder mehrere Lösungsmittel, ausgewählt aus einem Kohlenwasserstofflösungsmittel, einem Etherlösungsmittel und einem Esterlösungsmittel, ist.

3. Verfahren gemäß Anspruch 1 oder 2, wobei der in Schritt (2) abdestillierte Ammoniak zur Wiederverwendung Schritt (1) zugeführt wird.

4. Verfahren gemäß einem der Ansprüche 1 bis 3, wobei das in Schritt (5) abdestillierte organische Lösungsmittel zur Wiederverwendung Schritt (3) zugeführt wird.

5. Verfahren gemäß einem der Ansprüche 1 bis 4, wobei das in Schritt (5) abdestillierte Wasser zur Wiederverwendung Schritt (4) zugeführt wird.

6. Verfahren gemäß einem der Ansprüche 1 bis 5, wobei das in Schritt (6) abdestillierte Cyclohexanon zur Wiederverwendung Schritt (1) zugeführt wird.

7. Verfahren gemäß einem der Ansprüche 1 bis 6, wobei Schritt (4) die folgenden Schritte (4a) und (4b) umfasst:
(4a) einen Schritt des Mischens der in Schritt (3) erhaltenen organischen Schicht mit einer eine Base enthaltenden wässrigen Lösung, gefolgt von Trennen des Gemisches in eine organische Schicht und eine wässrige Schicht, und
(4b) einen Schritt des Mischens der in Schritt (4a) erhaltenen organischen Schicht mit Wasser, welches im Wesentlichen keine Base enthält, gefolgt von Trennen des Gemisches in eine organische Schicht und eine wässrige Schicht und die in Schritt (4b) erhaltene organische Schicht wird Schritt (5) unterzogen.

8. Verfahren gemäß Anspruch 7, wobei die in Schritt (4b) erhaltene wässrige Schicht mit einer Base gemischt wird und die resultierende, eine Base enthaltende wässrige Lösung zur Wiederverwendung Schritt (4a) zugeführt wird.

9. Verfahren gemäß Anspruch 7 oder 8, wobei das in Schritt (5) abdestillierte Wasser zur Wiederverwendung Schritt (4b) zugeführt wird.

## Revendications

1. Procédé de production d'une oxime de cyclohexanone, comportant les étapes (1) à (6) suivantes :
(1) une étape consistant à faire réagir de la cyclohexanone, du peroxyde d'hydrogène et de l'ammoniac en présence d'un catalyseur de titanosilicate pour donner une solution de réaction contenant de l'oxime de cyclohexanone, de l'eau, de l'ammoniac qui n'a pas réagi et de la cyclohexanone qui n'a pas réagi,
(2) une étape consistant à soumettre la solution de réaction obtenue à l'étape (1) à une distillation pour distiller de l'ammoniac et pour obtenir un résidu contenant de l'oxime de cyclohexanone, dé l'eau et de la cyclohexanone,
(3) une étape consistant à mélanger le résidu obtenu à l'étape (2) avec un solvant organique, opération suivie par une séparation d'un mélange en une couche organique et en une couche aqueuse,
(4) une étape consistant à mélanger la couche organique obtenue à l'étape (3) avec de l'eau, opération suivie d'une séparation d'un mélanger en une couche organique et en une couche aqueuse,
(5) une étape consistant à soumettre la couche organique obtenue à l'étape (4) à une distillation pour distiller le solvant organique et l'eau, et pour obtenir un résidu contenant de l'oxime de cyclohexanone et de la cylohexanone, et
(6) une étape consistant à soumettre le résidu obtenu à l'étape (5) à une distillation pour distiller de la cyclohexanone, et pour obtenir un résidu contenant de l'oxime de cyclohexanone.

2. Procédé selon la revendication 1, dans lequel le solvant organique utilisé dans l'étape (3) est un ou plusieurs solvants sélectionnés parmi un solvant d'hydrocarbure, un solvant d'éther, et un solvant d'ester.

3. Procédé selon la revendication 1 ou 2, dans lequel l'ammoniac distillé à l'étape (2) est réutilisé à l'étape (1).

4. Procédé selon l'une quelconque des revendications 1 à 3, dans lequel le solvant organique distillé à l'étape (5) est réutilisé à l'étape (3).

5. Procédé selon l'une quelconque des revendications 1 à 4, dans lequel l'eau distillée à l'étape (5) est réutilisée à l'étape (4).

6. Procédé selon l'une quelconque des revendications 1 à 5, dans lequel la cyclohexanone distillée dans l'étape (6) est réutilisée à l'étape (1).

7. Procédé selon l'une quelconque des revendications 1 à 6, dans lequel l'étape (4) comporte les étapes (4a) et (4b) suivantes :
(4a) une étape consistant à mélanger la couche organique obtenue à l'étape (3) avec une solution aqueuse contenant une base, opération suivie d'une séparation d'un mélange en une couche organique et en une couche aqueuse, et
(4b) une étape consistant à mélanger la couche organique obtenue à l'étape (4a) avec de l'eau ne contenant sensiblement pas une base, opération suivie d'une séparation d'un mélange en une couche organique et en une couche aqueuse, et
la couche organique obtenue à l'étape (4b) est soumise à l'étape (5).

8. Procédé selon la revendication 7, dans lequel la couche aqueuse obtenue à l'étape (4b) est mélangée avec une base, et la solution aqueuse résultante contenant une base est réutilisée à l'étape (4a).

9. Procédé selon la revendication 7 ou 8, dans lequel l'eau distillée à l'étape (5) est réutilisée à l'étape (4b).
